(19) Europäisches Patentamt
European Patent Office
Office européen des brevets

(11) Veröffentlichungsnummer: **0 150 404**
**B1**

(12) # EUROPÄISCHE PATENTSCHRIFT

(45) Veröffentlichungstag der Patentschrift: **26.11.86**

(21) Anmeldenummer: **84115521.1**

(22) Anmeldetag: **15.12.84**

(51) Int. Cl.⁴: **C 07 C 49/175**, C 07 C 45/63, C 07 C 45/39, C 07 D 233/60, C 07 D 249/02

(54) **Alpha-Halogen-phenoxyalkylketone, Phenoxyalkylketone, Verfahren zu ihrer Herstellung, sowie ihre Verwendung als Zwischenprodukte zur Herstellung von Fungiziden.**

(30) Priorität: **12.01.84 DE 3400829**

(43) Veröffentlichungstag der Anmeldung: **07.08.85 Patentblatt 85/32**

(45) Bekanntmachung des Hinweises auf die Patenterteilung: **26.11.86 Patentblatt 86/48**

(84) Benannte Vertragsstaaten: **AT BE CH DE FR GB IT LI NL SE**

(56) Entgegenhaltungen:
**DE-A-3 019 049**
**DE-A-3 209 431**
**DE-A-3 222 221**
**DE-B-2 716 896**

**ORGANIKUM, ORGANISCH-CHEMISCHES GRUNDPRAKTIKUM, 4. AUFLAGE, 1964 VEB DEUTSCHER VERLAG DER WISSENSCHAFTEN, Berlin**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch- Strasse 38, D-6700 Ludwigshafen (DE)**

(72) Erfinder: **Buschmann, Ernst, Dr., Georg- Ludwig-Krebs- Strasse 10, D-6700 Ludwigshafen (DE)**
Erfinder: **Sauter, Hubert, Dr., Neckarpromenade, D-6800 Mannheim 1 (DE)**

**EP 0 150 404 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft alpha-Halogen-phenoxyalkylketone, Phenoxyalkylketone, Verfahren zu ihrer Herstellung sowie ihre Verwendung als Zwischenprodukte zur Herstellung von Fungiziden.

alpha-Halogenketone sind bekannt und können durch alpha-Halogenierung der entsprechenden Ketone hergestellt werden. Problematisch sind dabei allerdings Halogenierungen in Gegenwart eines elektronenreichen aromatischen Substituenten z.B. eines Aryloxyrestes (Geza Zemplén u. Rezsö Bogner, Chem. Ber. 76, 452 (1943)). Das Halogenierungsmittel greift dabei zunächst den elektronenreichen aromatischen Kern an, und es entstehen unerwünschte Kernhalogenierungsprodukte.

Es wurde nun gefunden, daß alpha-Halogen-phenoxyalkylketone der Formel 1

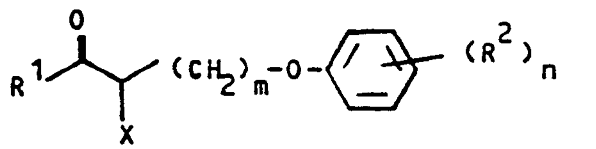

(I)

in welcher

$R^1$ tert.-Butyl, halogeniertes tert.-Butyl,

$R^2$ Halogen, einen Alkylrest mit 1 bis 4 Kohlenstoffatomen, einen Trifluormethylrest, eine Alkoxygruppe mit 1 bis 4 Kohlenstoffatomen oder Phenyl

X Cl oder Br,

m 2 bis 5 und

n 0 bis 5 bedeuten,

erhalten werden, wenn man Phenoxyalkylketone der Formel II

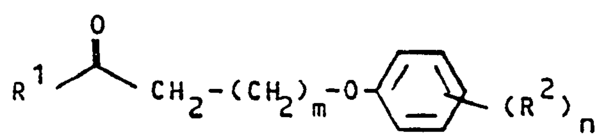

(II)

in welcher $R^1$, $R^2$, m und n die oben genannten Bedeutungen haben mit Halogenierungsmitteln umsetzt.

Überraschend führt die Halogenierung der Phenoxyalkylketone der Formel II zunächst nicht wie erwartet zur Halogenierung des aromatischen Rings, sondern zur alpha-Halogenierung der Ketofunktion und damit zu den alpha-Halogen-phenoxyalkylketonen der Formel I.

$R^1$ bedeutet beispielsweise tert.-Butyl, $FCH_2(CH_3)_2C$, $(FCH_2)_2CH_3C$, $(FCH_2)_3C$, $ClCH_2(CH_3)_2C$,

$R^2$ steht beispielsweise für Fluor, Chlor, Brom, Jod, Methyl, Ethyl, tertiär-Butyl, Isopropyl, Methoxy, Ethoxy, Phenyl, Trifluormethyl.

Die alpha-Halogenphenoxyalkylketone der Formel 1 sind wertvolle Zwischenprodukte für die Synthese der alpha-Azolylphenoxyalkylketone der Formel IV

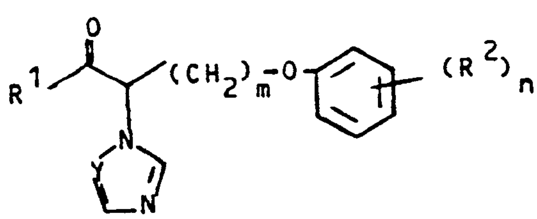

(IV)

in der $R^1$, $R^2$, m und n die oben angegebenen Bedeutungen haben und in der Y für CH und N steht. Die alpha-Azolylphenoxyalkylketone sind als fungizide Wirkstoffe in DE-OS 30 19 049 und DE-OS 32 09 431 beschrieben. Ausgangsverbindungen für die neuen alpha-Halogen-phenoxyalkylketone sind die Phenoxyalkylketone der Formel II, die wie in Schema 1 beschrieben, mit bekannten Reaktionen hergestellt werden können, beispielsweise durch Oxidation der entsprechenden Phenoxylakylalkohole der Formel III

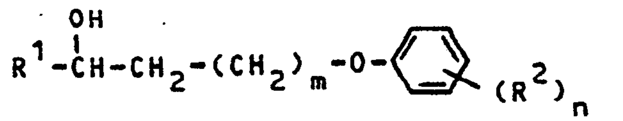

(III)

in der $R^1$, $R^2$, m und n die oben genannten Bedeutungen haben, mit Natriumbichromat und Schwefelsäure.

**Schema 1**

Herstellung der Phenoxyalkylketoné der Formel II

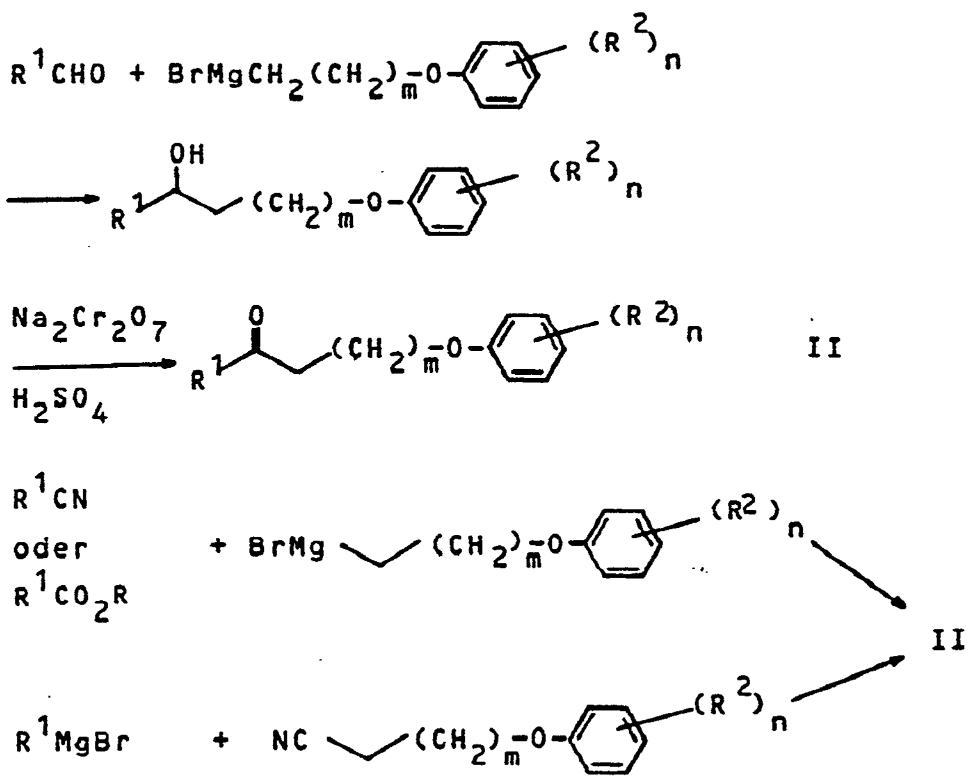


Halogenierung der Phenoxyalkylketone der Formel II führt zu den neuen alpha-Halogenphenoxyalkylketonen. In die Lösung des Phenoxyalkylketons der Formel II in einem geeigneten Lösungsmittel wird das Halogenierungsmittel zugetropft oder eingegast.

Geeignete Lösungsmittel sind beispielsweise: $CCl_4$, $CHCl_3$, $CH_2Cl_2$, Toluol, Benzol, Diisopropylether, Diethylether, $CH_3CO_2H$, $CS_2$

Als Halogenierungsmittel können beispielsweise Verwendung finden: Chlor, Brom, N-Br-Succinimid, Bromkomplexe von Pyridiniumsalzen, Sulfurylchlorid.

Die Halogenierungsreaktion kann durch Katalysatoren wie Lewis-Säuren (z.B. HBr, $AlCl_3$) und Radikalstarter (z.B. Azobisisobutyronitril) beschleunigt werden.

Die Umsetzung erfolgt beispielsweise bei einer Temperatur von 15 bis 30°C. Zweckmäßig verwendet man das Halogenierungsmittel im Überschuß, beispielsweise 0,1 bis 10 % Überschuß.

**Vorschrift A**

Herstellung von 2,2-Dimethyl-8-phenoxy-octanol-3 (A)

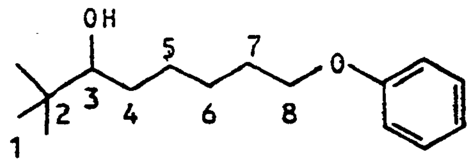

Zu 19,8 g Magnesiumspäne in 100 ml Ether (Diethylether) werden 200 g l-Brom-5-phenoxypentan, in 250 ml Ether gelöst, zugetropft. Nach Zugabe von 10 % der l-Brom-5-phenoxypentanlösung werden einige Tropfen Brom zugegeben. Nachdem die Grignardreaktion angesprungen ist wird weiter zugetropft. Anschließend wird 1/2 h zum Rückfluß erwärmt. Man tropft 60 g Pivalaldehyd in 200 ml Ether zu und erwärmt 2 h zum Rückfluß. Nach Hydrolyse mit gesättigter wäßriger $NH_4Cl$-Lösung wird das Produkt mit Ether extrahiert. Man trocknet über $Na_2SO_4$, engt ein und destilliert. Man erhält 105 g 2,2-Dimethyl-8-phenoxy-octanol-3 (A) Kp. 130 bis 142°/0,2 mbar.

**Beispiel 1**

2,2-Dimethyl-8-phenoxy-octanon-3 (H)

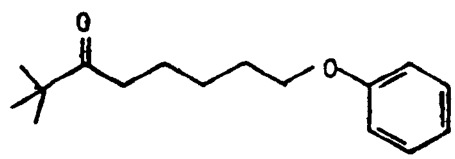

Zu 90 g (A) in 400 ml Ether wird bei 20 bis 25°C unter Eiskühlung zugetropft eine Lösung von 57 g $Na_2Cr_20_7.2H_20$ und 40 ml $H_2SO_4$ (konz.) in 0,5 1 $H_20$. Man rührt 2 h bei 25°C, trennt die etherische Phase ab und extrahiert noch zweimal mit Ether. Die vereinigten Etherphasen werden mit wäßriger $NaHCO_3$-Lösung gewaschen, über $Na_2SO_4$ getrocknet, eingeengt und destilliert. Man erhält 60 g (B) Kp. 135-137°/0,2 mbar (Verbindung 1.4).

**Beispiel 2**

4-Brom-2,2-dimethyl-8-phenoxy-octanon-3 (C)

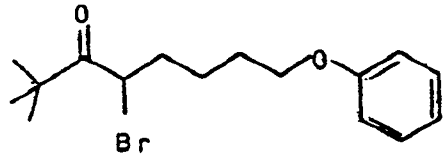

Zu 48 g (B) in 500 ml Diethylether werden bei Raumtemperatur (20°C) 32 g Brom zugetropft. Man rührt 2 h nach, versetzt mit 1 l Eiswasser und extrahiert das Produkt mit Ether. Die etherische Phase wird mit wäßriger $Na_2CO_3$-Lösung und mit Wasser gewaschen. Man trocknet über $Na_2SO_4$ und engt die Lösung ein. Vor dem Einengen gibt man noch eine Spatelspitze $Na_2CO_3$ zur Stabilisierung des Produktes zu. Das nach dem Einengen erhaltene Produkt kann ohne weitere Reinigung mit 1,2,4-Triazol oder Imidazol weiter ungesetzt werden. (C) zeigt das folgende NMR-Spektrum (δ-Werte, $CDCl_3$): 6,72-7,48 (m, 5H), 4,70 (t, J = 8Hz, 1H), 4,00 (t, J = 8 Hz, 2H), 1,48-2,68 (m, 6H), 1,31 (s, 9H) (Verbindung 2.6).

**Vorschrift B**

2,2-Dimethyl-8-phenoxy-4-(1,2,4-triazol-l-yl)-octanon-3 (D)

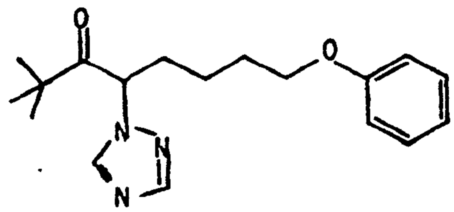

Zu einer Suspension von 51 g $Na_2CO_3$ und 33,2 g 1,2,4-Triazol in 600 ml Ethanol werden unter Rückfluß 77 g (C) in 200 ml Ethanol zugetropft. Man erwärmt weitere 3 h zum Rückfluß, engt ein, nimmt auf mit $CH_2Cl_2/H_2O$, wäscht mit Wasser, trocknet über $Na_2SO_4$, engt ein und destilliert. Man erhält 26 g (D) Kp. 170-178°/0,1 mbar.

Entsprechend Beispiel 1 können die Phenoxyalkylketone der folgenden Tabelle hergestellt werden:

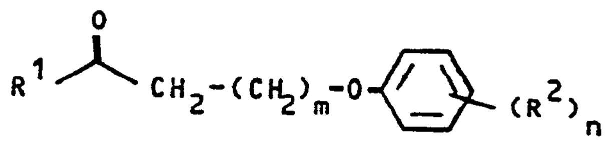

| Beispiel Nr. | $R^1$ | $(R^2)_n$ | m | Sdp °C/mbar |
|---|---|---|---|---|
| 1.1 | tert.Butyl | H | 3 | 113°/0,2 |
| 1.2 | tert.Butyl | H | 2 | |
| 1.3 | tert.Butyl | H-Cl | 2 | |
| 1.4 | tert.Butyl | H | 4 | 135-137°/0,2 |
| 1.5 | tert.Butyl | 2,4-$Cl_2$ | 4 | |
| 1.6 | tert.Butyl | 4-Cl | 4 | |
| 1.7 | tert.Butyl | 4-F | 4 | |
| 1.8 | tert.Butyl | 2-F | 4 | |

Entsprechend dem Beispiel 2 können die alpha-Halogenphenoxyalkylketone der folgenden Tabelle hergestellt werden:

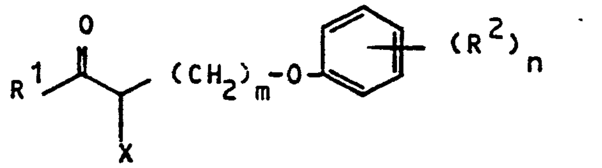

| Beispiel Nr. | $R^1$ | $(R^2)_n$ | m | X | NMR-Spektrum δ-Werte, $CDCl_3$ |
|---|---|---|---|---|---|
| 2.1 | tert.-Butyl | H | 3 | Br | 6,6-7,4 (m, 5H); 4,70 (t, J = 8Hz, 1H); 3,86 (t, J = 8Hz, 2H); 1,48-2,38 (m, 2H); 1,17 (s, 9H) |
| 2.2 | tert.-Butyl | H | 2 | Br | |
| 2.3 | tert.-Butyl | H | 2 | Cl | |
| 2.4 | tert.-Butyl | 4-Cl | 2 | Br | |
| 2.5 | tert.-Butyl | H | 3 | Cl | |
| 2.6 | tert.-Butyl | H | 4 | Br | (Beispiel 2) |
| 2.7 | tert.-Butyl | H | 4 | Cl | |
| 2.8 | tert.-Butyl | $2,4-Cl_2$ | 4 | Br | |
| 2.9 | tert.-Butyl | 4-Cl | 4 | Br | |
| 2.10 | tert.-Butyl | 4-F | 4 | Br | |
| 2.11 | tert.-Butyl | 2-F | 4 | Br | |
| 2.12 | tert.-Butyl | 2-F | 4 | Cl | |

Wie in Vorschrift B beschrieben, kann man aus alpha-Halogenphenoxyalkylketonen die entsprechenden Azolylphenoxyalkylketone der Formel IV herstellen:

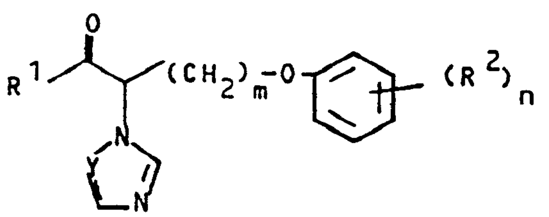

(IV)

| Vorschrift B Nr. | $R^1$ | $(R^2)_n$ | m. | Y |
|---|---|---|---|---|
| 3.1 | tert.-Butyl | H | 3 | N |
| 3.2 | tert.-Butyl | H | 3 | CH |
| 3.3 | tert.-Butyl | H | 2 | N |
| 3.4 | tert.-Butyl | H | 2 | CH |
| 3.5 | tert.-Butyl | H | 4 | N |
| 3.6 | tert.-Butyl | H | 4 | CH |
| 3.7 | tert.-Butyl | 4-Cl | 2 | N |
| 3.8 | tert.-Butyl | 4-Cl | 2 | N |
| 3.9 | tert.-Butyl | $2,4-Cl_2$ | 4 | N |
| 3.10 | tert.-Butyl | $2,4-Cl_2$ | 4 | CH |
| 3.11 | tert.-Butyl | 2-F | 4 | N |
| 3.12 | tert.-Butyl | 2-F | 4 | CH |
| 3.13 | tert.-Butyl | 4-F | 4 | N |
| 3.14 | tert.-Butyl | 4-F | 4 | CH |

**Patentansprüche**

für die Vertragsstaaten: BE, CH, DE, FR, GB, IT, LI, NL, SE.

1. alpha-Halogen-phenoxyalkylketon der Formel 1

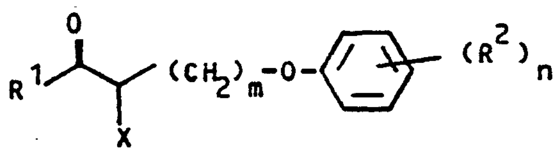 (I)

in welcher
$R^1$ tert.-Butyl, halogeniertes tert.-Butyl,
$R^2$ Halogen, einen Alkylrest mit 1 bis 4 Kohlenstoffatomen, einem Trifluormethylrest, eine Alkoxygruppe mit 1 bis 4 Kohlenstoffatomen oder Phenyl
X Cl oder Br,
m 2 bis 5 und
n 0 bis 5 bedeuten.

2. Phenoxyalkylketon der Formel II

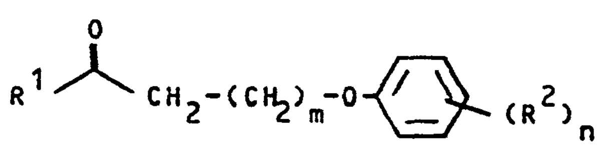 (II)

in welcher $R^1$, $R^2$, m und n die im Anspruch 1 genannten Bedeutungen haben.

3. Verfahren zur Herstellung der alpha-Halogenphenoxyalkylketone der Formel I, gemäß Anspruch I, dadurch gekennzeichnet, daß man ein Phenoxyalkylketon der Formel II

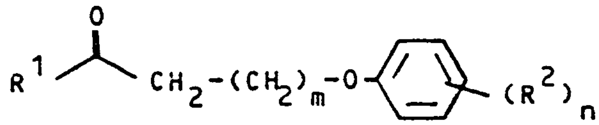 (II)

in der $R^1$, $R^2$, m und n die im Anspruch 1 genannten Bedeutungen haben, mit Halogenierungsmitteln umsetzt.

4. Verwendung der alpha-Halogen-phenoxyalkylketone der Formel 1 gemäß Anspruch 1 zur Herstellung von fungiziden Azolylphenoxyalkylketonen, dadurch gekennzeichnet, daß man die alpha-Halogen-phenoxyalkylketone mit Triazol oder Imidazol umsetzt.

5. Verfahren zur Herstellung der Phenoxyalkylketone der Formel II gemäß Anspruch 2, dadurch gekennzeichnet, daß man einen Phenoxyalkylalkohol der Formel III

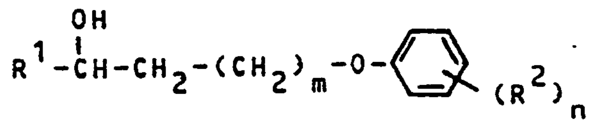 (III)

in der $R^1$, $R^2$, m und die im Anspruch 1 genannten Bedeutungen haben, mit Natriumbichromat und Schwefelsäure oxidiert.

6. 4-Brom-2,2;dimethyl-7-phenoxy-heptanon-3.

7. 4-Brom-2,2-dimethyl-8-phenoxy-octanon-3.

**Patentansprüche**

für den Vertragsstaat AT.

1. Verfahren zur Herstellung von alpha-Halogenphenoxyalkylketonen der Formel I

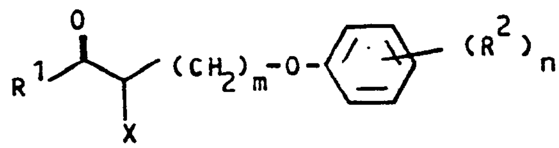 (I)

in welcher
$R^1$ tert.-Butyl, halogeniertes tert.-Butyl,
$R^2$ Halogen, einen Alkylrest mit 1 bis 4 Kohlenstoffatomen, einem Trifluormethylrest, eine Alkoxygruppe mit 1 bis 4 Kohlenstoffatomen oder Phenyl
X Cl oder Br,
m 2 bis 5 und
n 0 bis 5 bedeuten,
dadurch gekennzeichnet, daß man ein Phenoxyalkylketon der Formel II

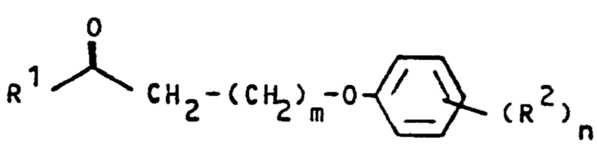 (II)

in der $R^1$, $R^2$, m und n die oben genannten Bedeutungen haben, mit Halogenierungsmitteln umsetzt.

2. Verwendung der alpha-Halogen-phenoxyalkylketone der Formel 1 gemäß Anspruch 1 zur Herstellung von fungiziden Azolylphenoxyalkylketonen, dadurch gekennzeichnet, daß man die alpha-Halogen-phenoxyalkylketone mit Triazol oder Imidazol umsetzt.

3. Verfahren zur Herstellung der Phenoxyalkylketone der Formel II

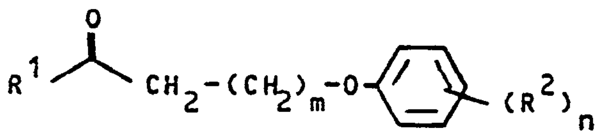

(II)

in welcher $R^1$, $R^2$, m und n die im Anspruch 1 genannten Bedeutungen haben, dadurch gekennzeichnet, daß man einen Phenoxyalkylalkohol der Formel III

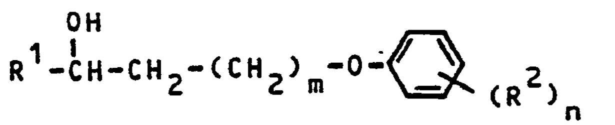

(III)

in der $R^1$, $R^2$, m und die im Anspruch 1 genannten Bedeutungen haben, mit Natriumbichromat und Schwefelsäure oxidiert.

**Claims**

: for the Contracting States: BE CH DE FR GB IT LI NL SE

1. An alpha-halophenoxyalkyl ketone of the formula I

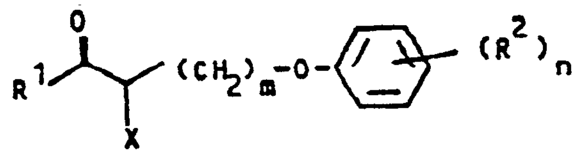

(I) ,

where
$R^1$ is tert.-butyl or halogenated tert.-butyl,
$R^2$ is halogen, alkyl of 1 to 4 carbon atoms, trifluoromethyl, an alkoxy group of 1 to 4 carbon atoms, or phenyl,
X is Cl or Br,
m is 2 to 5, and
n is 0 to 5.

2. A phenoxyalkyl ketone of the formula II

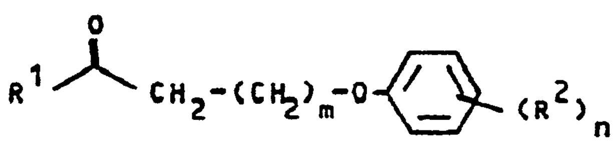

(II) ,

where $R^1$, $R^2$, m and n have the meanings given in claim 1.

3. A process for the preparation of an alpha-halophenoxyalkyl ketone of the formula I as claimed in claim 1, characterized in that a phenoxyalkyl ketone of the formula II

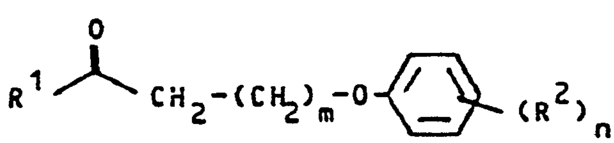

(II)

where $R^1$, $R^2$, m and n have the meanings given in claim 1, is reacted with a halogenating agent.

4. The use of an alpha-halophenoxyalkyl ketone of the formula I as claimed in claim 1 for the preparation of a fungicidal azolylphenoxyalkyl ketone, characterized in that the alpha-halophenoxyalkyl ketone is reacted with triazole or imidazole.

5. A process for the preparation of a phenoxyalkyl ketone of the formula II as claimed in claim 2, characterized in that a phenoxyalkyl alcohol of the formula III

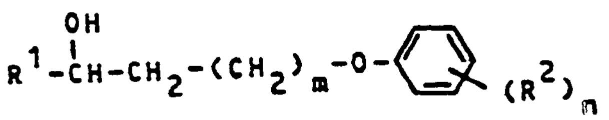 (III),

where $R^1$, $R^2$, m and n have the meanings given in claim 1, is oxidized with sodium bichromate and sulphuric acid.

6. 4-Bromo-2,2-dimethyl-7-phenoxyheptan-3-one.

7. 4-Bromo-2,2-dimethyl-8-phenoxyoctan-3-one.

**Claims**

: for the Contracting State AT

1. A process for the preparation of an alpha-halophenoxyalkyl ketone of the formula I

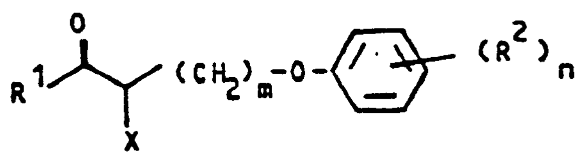 (I),

where
$R^1$ is tert.-butyl or halogenated tert.-butyl,
$R^2$ is halogen, alkyl of 1 to 4 carbon atoms, trifluoromethyl, an alkoxy group of 1 to 4 carbon atoms, or phenyl,
X is Cl or Br,
m is 2 to 5, and
n is 0 to 5,
characterized in that a phenoxyalkyl ketone of the formula II

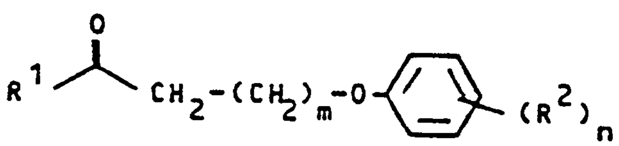 (II),

where $R^1$, $R^2$, m and n have the abovementioned meanings, is reacted with a halogenating agent.

2. The use of an alpha-halophenoxyalkyl ketone of the formula I as claimed in claim 1 for the preparation of a fungicidal azolylphenoxyalkyl ketone, characterized in that the alpha-halophenoxyalkyl ketone is reacted with triazole or imidazole.

3. A process for the preparation of a phenoxyalkyl-ketone of the formula II

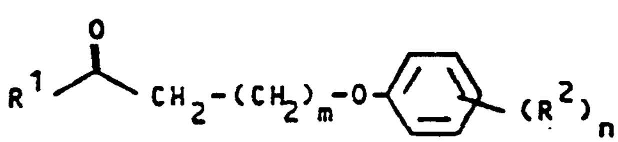 (II),

where $R^1$, $R^2$, m and n have the meanings given in claim 1, characterized in that a phenoxyalkyl alcohol of the formula III

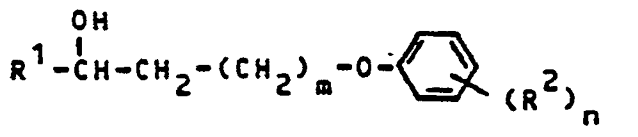

(III),

where R¹, R², m and n have the meanings given in claim 1, is oxidized with sodium bichromate and sulphuric acid.

**Revendications**

: pour les Etats contractants: BE CH DE FR GB IT LI NL SE

1. α-halogémophénoxyalkylcétions de formule I

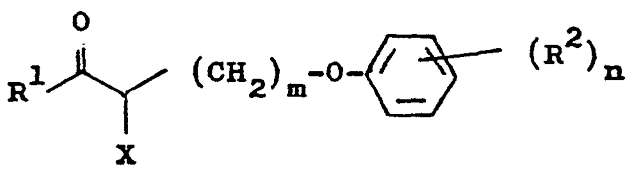

(I)

dans laquelle

$R^1$ représente tert-butyle, tert-butyle halogéné

$R^2$ halogène, un reste alkyle ayant 1 à 4 atomes de carbone, un reste trifluorométhyle, un groupe alcoxy ayant 1 à 4 atomes de carbone ou phényle,

X Cl ou Br

m 2 à 5 et

n 0 à 5

2. Phénogyalkylcétone de formule II

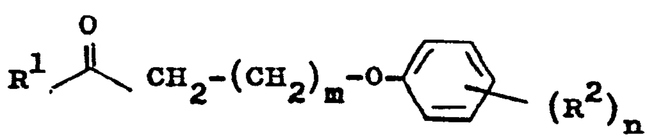

(II)

dans laquelle $R^1$, $R^2$, m et n ont les significations indiquées dans la revendication 1.

3. Procédé de préparation des α-halogénophénoxyalkylcétones de formule I selon la revendication 1, caractérisé par le fait que l'on fait réagir une phénoxyalkylcétone de formule II

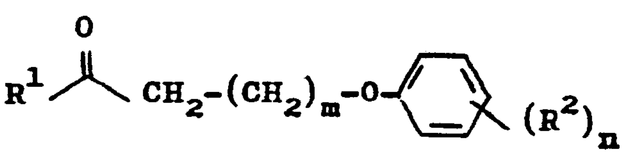

(II)

dans laquelle $R^1$, $R^2$, m et n ont les significations indiquées dans la revendication 1, avec des agents d'halogénation.

4. Utilisation des α-halogénophénolyalkylcétones de formule 1 selon la revendication 1 pour la préparation d'azolylphénoxyalkylcétones fongicides, caractérisée par le fait qu'on fait réagir les α-halogénophénoxyalkylcétones avec du triazole ou imidazole.

5. Procédé de préparation des phénoxyalkylcétones de formule II selon la revendication 2, caractérisé par le fait que l'on oxyde un phénoxyalkylalcool de formule III

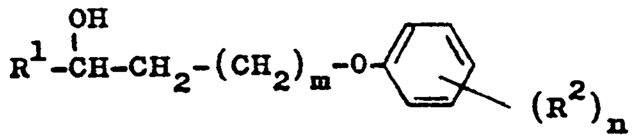

(III)

dans laquelle $R^1$, $R^2$, m et n ont les significations indiquées dans la revendication 1, avec du bichromate de sodium et de l'acide sulfurique.

6. 4-bromo-2,2-diméthyl-7-phénoxy-heptanone-3.

7. 4-bromo-2,2-diméthyl-8-phénoxy-octanone-3.

**Revendications**

: pour l'Etat contractant AT

1. Procédé de préparation de α-halogénophénoxyalkylcétones de formule I

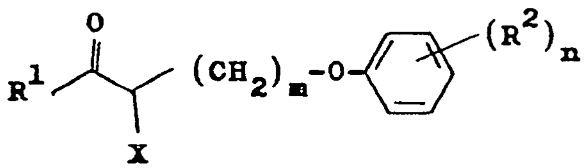

(I)

dans laquelle

$R^1$ représente tert-butyle, tert-butyle halogéné

$R^2$ halogène, un reste alkyle ayant 1 à 4 atomes de carbone, un reste trifluorométhyle, un groupe alcoxy ayant 1 à 4 atomes de carbone ou phényle,

X Cl ou Br

m 2 à 5 et

n 0 à 5

caractérisé par le fait que l'on fait réagir une phénoxyalkylcétone de formule II

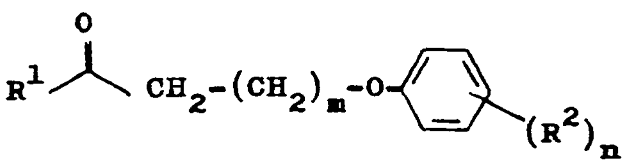

(II)

dans laquelle $R^1$, $R^2$, m et n ont les significations données ci-dessus, avec des agents d'halogénation.

2. Utilisation des α-halogénophénoxyalkylcétones de formule I selon la revendication 1 pour la préparation d'azolylphénoxyalkylcétones fongicides, caractérisée par le fait qu'on fait réagir les α-halogénophénoxyalkylcétones avec du triazole ou imidazole.

3. Procédé de préparation de phénoxyalkylcétones de formule II

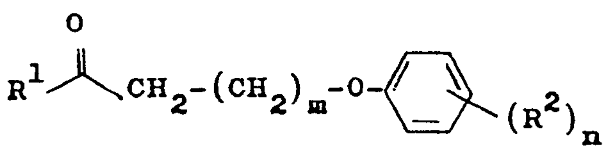

(II)

dans laquelle $R^1$, $R^2$, m et n ont les significations données dans la revendication 1, caractérisé par le fait que l'on oxyde un phénoxyalkylalcool de formule III

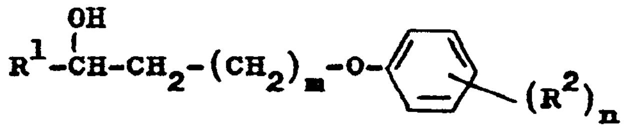 (III)

dans laquelle $R^1$, $R^2$, m et n ont les significations indiquées dans la revendication 1, avec du bichromate de sodium et de l'acide sulfurique.